(19) Europäisches Patentamt
European Patent Office
Office européen des brevets

(11) EP 2 042 159 B1

(12) EUROPEAN PATENT SPECIFICATION

(45) Date of publication and mention
of the grant of the patent:
**21.09.2016 Bulletin 2016/38**

(51) Int Cl.:
*A61K 8/92* (2006.01)   *A61K 8/25* (2006.01)
*A61K 8/89* (2006.01)   *A61Q 1/12* (2006.01)
*A61Q 19/00* (2006.01)

(21) Application number: **07744330.7**

(22) Date of filing: **29.05.2007**

(86) International application number:
**PCT/JP2007/060911**

(87) International publication number:
**WO 2007/139114 (06.12.2007 Gazette 2007/49)**

(54) **AGENT FOR IMPROVING FINE WRINKLES**

MITTEL ZUR VERBESSERUNG FEINER FÄLTCHEN

AGENT POUR LA RÉDUCTION DES RIDES FINES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **01.06.2006 JP 2006153201**

(43) Date of publication of application:
**01.04.2009 Bulletin 2009/14**

(73) Proprietor: **Shiseido Company, Limited Tokyo 104-8010 (JP)**

(72) Inventors:
• **SUDA, Mari**
**Yokohama-shi, Kanagawa 224-8558 (JP)**
• **TAKAHASHI, Akiko**
**Yokohama-shi, Kanagawa 224-8558 (JP)**
• **OKAMOTO, Tohru**
**Yokohama-shi, Kanagawa 224-8558 (JP)**
• **MATSUNO, Fumio**
**Yokohama-shi, Kanagawa 224-8558 (JP)**

(74) Representative: **Scholz, Volker et al
Boehmert & Boehmert
Anwaltspartnerschaft mbB
Patentanwälte Rechtsanwälte
Pettenkoferstrasse 20-22
80336 München (DE)**

(56) References cited:
**EP-A1- 1 491 178      EP-A1- 1 627 629
EP-A1- 1 839 647      WO-A1-02/05751
WO-A1-03/059315      JP-A- 2000 026 232
JP-A- 2003 002 814    JP-A- 2003 026 608
JP-A- 2005 187 426    US-A- 5 919 468
US-A- 5 945 095**

• **DATABASE CA [Online] CHEMICAL ABSTRACTS SERVICE, COLUMBUS, OHIO, US; 25 January 1960 (1960-01-25), Kuroda, Akihiro et al.: "Lip cosmetic bases containing silicone elastomers, their application method, and cosmetic sets" XP002606682 retrieved from STN Database accession no. 138:44515 & JP 2003 002814 A 8 January 2003 (2003-01-08)**
• **DATABASE CA [Online] CHEMICAL ABSTRACTS SERVICE, COLUMBUS, OHIO, US; 25 January 1960 (1960-01-25), Fukuda, Nobuo et al.: "Anti-itching topical compositions containing anti-inflammatories and vaseline" XP002606683 retrieved from STN Database accession no. 138:112483 & JP 2003 026608 A 29 January 2003 (2003-01-29)**

EP 2 042 159 B1

**Description**

Technical Field

This invention relates to the use of an oil preparation and a cosmetic method for diminishing fine wrinkles.

Background Art

In proportion as persons grow old, wrinkles increase as one of skin aging phenomena. In accordance with wrinkle occurrence sites, wrinkle occurrence mechanisms, and the like, wrinkles maybe roughly classified into large wrinkles, fine wrinkles, and reticulation. The large wrinkles are the deep wrinkles occurring at the backs of the necks, and the like, primarily due to photoaging. The fine wrinkles are the comparatively shallow wrinkles occurring about the eyes or the mouths. The reticulation is the pleat-like wrinkles occurring at unexposed body regions, such as abdomens of old persons.

Recently, females of middle or advanced age take an increasing interest in beauty culture and have an increasing interest in the fine wrinkles occurring about the eyes or the mouths, which fine wrinkles appear due to lowering of water retention capability of the corneal layer of the epidermis with age or due to a decrease in sebum caused to occur by lowering of secretion of epidermal lipid with age.

Cosmetic preparations for the fine wrinkles may be roughly classified into make-up preparations, which make the fine wrinkles visually imperceptible, and skin care preparations, which have the improving effects when being used successively.

The make-up preparations make the fine wrinkles visually imperceptible by smoothing the unevenness of the skin or correcting the skin color. For example, a preparation for making the fine wrinkles imperceptible by blurring the unevenness of the fine wrinkles by light diffusing effects of spherical particles (e.g., patent literature 1 or 2), a preparation for smoothing the fine wrinkles by protrusion and recess filling-up effects of a wax, a polymer gel, or the like (e.g., patent literature 3), and a preparation utilizing both of the aforesaid preparations have been reported. The make-up preparations make the fine wrinkles visually imperceptible, but do not have the effects of actually improving the fine wrinkles.

The skin care preparations improve the fine wrinkles by being blended with various medicines, moisturizing agents, and the like. For example, a preparation blended with medicines, such as a vitamin C derivative, vitamin A, and a vitamin A derivative, (e.g., patent literature 4), and a preparation blended with a moisturizing agent, such as glycerol, sorbitol, or a vegetable liquid extract, collagen, or the like, have been known. However, the skin care cosmetic preparations are markedly expensive, and it is necessary to use them successively for a certain period of time in order for the fine wrinkle improving effects to be obtained.

Patent literature 1: Japanese Unexamined Patent Publication (Kokai) No. 2002-047138
Patent literature 2: Japanese Unexamined Patent Publication (Kokai) No. 2003-012461
Patent literature 3: Japanese Unexamined Patent Publication (Kokai) No. 2000-016919
Patent literature 4: Japanese Unexamined Patent Publication (Kokai) No. 2002-080338

JP 2000 026232 A refers to solid make-up cosmetics which are used for hiding wrinkles. The document describes a solid cosmetic which comprises at least 40 wt% of oil based constituents having a refractive index not less than 1.39 and less than 1.45, 10-50 wt% of colorless spherical or amorphous fine particles having a refractive index from 1.45 to 1.55 and an average particle diameter from 2 to 20 $\mu$m, and a wax, in which the content of oil based constituents having refractive indexes from 1.45 to 1.55 is not more than 20 wt%.

JP 2003 002814 A describes foundations for lips which have wrinkle concealing effects, comprising (A) a paste material obtained by kneading or crushing a mixture of a silicone elastomer and one or more liquid oil constituents selected from ester oils, hydrocarbon oils, higher alcohols, vegetable oils and animal oils, (B) platy particles having an average particle diameter from 1 to 50 $\mu$m, and (C) an oil constituent.

The invention of JP 2003 026608 A relates to vaseline-based topical compositions for control of itching in dermatitis without tacky feel. The composition comprises (a) 30-85% of a white vaseline, (b) 2-25 wt% of powders, (c) 0.005-10 wt% of phosphorylcoline-containing copolymers, and (d) 0.01-3 wt% of anti-inflammatories or disinfectants.

EP 1 839 647 A1 discloses an oil based composition for external use on skin for enhancing percutaneous absorption of a water-soluble agent contains 50% by mass to 95% by mass of an oil constituent, which contains 10% by mass to 100% by mass of a solid or a semisolid oil constituent, and 5% by mass to 50% by mass of particles. The composition has occlusivity of at least 50% and is adapted for use such that, after a preparation for external use on a skin, which preparation contains a water-soluble agent, has been apply onto skin, the composition may be applied onto the preparation for external use on skin, which preparation has been applied onto the skin. A method of enhancing percutaneous absorption of a water-soluble agent utilizes the composition.

US 5,945,095 A relates to a composition, in particular a cosmetic, dermatological, hygiene or pharmaceutical composition, to care for and/or make-up the skin, which composition may be in form of a compact powder or a cast product, comprising a dispersion of polymer particles in a fatty substance. Furthermore, the invention relates to the use of this dispersion in such a composition.

In WO 02/05751 A1 a composition for the topical application of vitamin C is described, comprising one or more lipid-soluble forms of vitamin C, one or more water-soluble forms of vitamin C and one or more $\alpha$-hydroxylated acids. The composition can also comprise an anhydrous gel, ethoxy diglycol, a lipid-soluble analog of pro-vitamin B-5, $\alpha$-bisabolol, and one or more forms of vitamin E.

US 5,919,468 A relates to the use of a partially crosslinked elastomeric solid organopolysiloxane in combination with a fatty phase for the preparation of a composition or in a composition for skin care or make-up for matting the skin, and to its various applications. The cosmetic compositions according to the invention are mild in application, easily spread, non-sticky and do not dry out the skin. They can be employed in particular for blurring out skin relief blemishes such as microreliefs, wrinkles or pores, while giving the skin a natural appearance.

EP 1 627 629 A1 provides a cosmetic, particularly skin cosmetic, excellent in imparting transparency to the skin, conditioning a skin texture and making wrinkles and hair follicles imperceptible when applied. The cosmetic comprises a powder of porous spherical silica having an average particle size from 3.0 to 20 $\mu$m, a maximum particle size of 50 $\mu$m or smaller, and a pore volume from 1.5 to 3.0 cm$^3$/g, wherein the powder is characterized in that the minimum value of dlog (storage elasticity)/dlog (shear stress) is -10 or more, when 40 g of squalane is added to 15 cm$^3$ of appearing volume of said powder of porous spherical silica, the resulting paste is charged in thickness of 2 mm between parallel plates of 2.5 cm diameter, one of the plates is subjected to an angular vibration to another in a frequency of 2 Hz, dynamic viscoelasticity is measured by increasing an average shear stress between the plates from 10 Pa to 10 kPa and correlation between lock (shear stress) versus lock (storage elasticity) is measured.

Disclosure of Invention

Problems Which the Invention Aims at Solving

The object of the present invention is to provide the use of an agent for improving fine wrinkles, which improves fine wrinkles efficiently within a short period of time without the necessity of expensive medicines, moisturizing agents, or the like, being blended, and which is low in cost and has good usability.

Another obj ect of the present invention is to provide a cosmetic method, which improves fine wrinkles efficiently within a short period of time without the necessity of expensive medicines, moisturizing agents, or the like, being used.

Means for Solving the Problems

The inventors have found that, in cases where an oil based preparation is used for diminishing fine wrinkles, which oil based preparation has occlusion characteristics of as high as at least 50%, is applied onto a skin, water within the skin is retained efficiently on the surface of the skin, and fine wrinkles are improved markedly within a short period of time, and that, in cases where 70% by mass to 85% by mass of an oil constituent, which contains a solid or semisolid non-polar hydrocarbon oil in a proportion of at least 30% by mass with respect to a total quantity of the oil constituent, and 15% by mass to 50% by mass of particles are blended together, the usability of the oil based preparation for diminishing fine wrinkles is improvedmarkedly, such that the occlusion characteristics of the oil based preparation for diminishing fine wrinkles may not be affected adversely. The present invention is based upon the findings described above.

Conventional oily base materials having high occlusion characteristics, such as petrolatum, exhibit bad spreadability. Also, when being coated onto the skin, the conventional oily base materials give a sticky feeling and oily sheen. The usability of the conventional oily base materials is thus markedly low. Such that the usability of the petrolatum, or the like, may be improved, various attempts have heretofore been made to blend various kinds of particles. However, with the conventional preparations for external use, wherein the particles are blended, paths allowing the passage of water are formed through the oil based system, and the occlusion characteristics become low. Therefore, water within the skin is not capable of being retained sufficiently on the skin surface, and the fine wrinkle improving effects are not capable of being obtained.

The present invention provides the use of an oil based preparation for diminishing fine wrinkles, wherein the oil based preparation containing:

i) 70% by mass to 85% by mass of an oil constituent, which contains a solid or semisolid non-polar hydrocarbon oil in a proportion of at least 30% by mass with respect to a total quantity of the oil constituent, and

ii) 15% by mass to 30% by mass of particles, which comprise elastic spherical silicone type particles and spherical silica particles,

wherein the oil based preparation has occlusion characteristics of at least 50%, and the proportion of water and water-soluble constituents contained in the oil based preparation is at most 1 % by mass.

The present invention also provides a cosmetic method for diminishing fine wrinkles, wherein an oil based preparation for external use for skins is applied onto a skin, the oil based preparation containing:

i) 70% by mass to 85% by mass of an oil constituent, which contains a solid or semisolid non-polar hydrocarbon oil in a proportion of at least 30% by mass with respect to a total quantity of the oil constituent, and

ii) 15% by mass to 30% by mass of particles, which comprise elastic spherical silicone type particles and spherical silica particles,

wherein the oil based preparation has occlusion characteristics of at least 50%, and the proportion of water and water-soluble constituents contained in the oil based preparation is at most 1 % by mass.

The term "occlusion characteristics" as used herein means the value calculated with the formula shown below and in accordance with a transepidermal water loss (TEWL), which is measured with a water loss meter at a stage one hour after a sample has been applied (at a rate of 2.5mg/cm$^2$) to an internal site of a human forearm.

$$\text{Occlusion characteristics (\%)}$$
$$= (1-\text{TEWL (with sample) / TEWL (without sample)}) \times 100$$

In the present invention, the particles contain spherical particles. The spherical particles are blended with the oil based preparation for use of the oil based preparation for diminishing fine wrinkles in accordance with the present invention, the coating and extension of the oil based preparation for diminishing fine wrinkles are capable of being performed smoothly, and the use feeling is capable of being improved. Also, the unevenness of the skin is capable of being blurred and rendered imperceptible by virtue of the light scattering effects of the spherical particles contained in the oil based preparation having been applied onto the skin. The oil based preparation for diminishing fine wrinkles is capable of being used at the time of skin care and at the time of make-up, and therefore the preparation having both of the fine wrinkle improving effects and the fine wrinkle concealing effects is capable of being furnished. Particularly, elastic spherical particles are free from a powdery feeling and are therefore preferable. Among the elastic spherical particles, elastic spherical silicone type particles having high effects of suppressing a sticky feeling are blended. Further, in cases where spherical silica particles are used in conjunction with the elastic spherical particles or the elastic spherical silicone type particles, the usability and the high-temperature vertical storage stability are capable of being enhanced.

From the view point of the occlusion characteristics, the oil constituent contains a solid or semisolid non-polar hydrocarbon oil constituent in a proportion of at least 30% by mass with respect to a total quantity of the oil constituent. In cases where the non-polar hydrocarbon oil constituent is blended at a high concentration, the occlusion characteristics are capable of being enhanced even further.

Also, from the viewpoint of the usability, the oil constituent should preferably contain a volatile oil constituent in a proportion falling within the range of 5% by mass to 50% by mass with respect to a total quantity of the oil constituent. In cases where the volatile oil constituent is blended, the coating and the extension are capable of being performed more easily, and the sticky feeling of the coated composition is capable of being improved even further.

Effects of the Invention

With the use of the oil based preparation for diminishing fine wrinkles in accordance with the present invention, which has the occlusion characteristics of at least 50%, water coming from the inside region of the skin is retained at the surface of the skin, and the skin is capable of being kept in a soft and full state. Therefore, the fine wrinkles are improved markedly within a short period of time without the necessity of expensive medicines, moisturizing agents, or the like, being used.

Also, the oil based preparation for diminishing fine wrinkles, which is used in accordance with the present invention contains 70% by mass to 85% by mass of the oil constituent, which contains the solid or semisolid non polar hydrocarbon oil in a proportion of at least 30% by mass with respect to a total quantity of the oil constituent, and 15% by the oil based preparation for diminishing fine wrinkles is applied onto the skin, the oil based preparation does not give the sticky feeling and the oily sheen and exhibits good characteristics of occluding the skin. Further, with the use of the oil based preparation in accordance with the present invention, which has markedly good spreadability, sufficient occlusion characteristics (and consequently sufficient effects of improving the fine wrinkles) are obtained in cases where a small quantity of the oil based preparation is used.

Best Mode for Carrying Out the Invention

Each of the oil based preparation, which is used for diminishing fine wrinkles in accordance with the present invention, is contained in the agent for improving fine wrinkles , and the oil based preparation for diminishing fine wrinkles which is employed in the cosmetic method in accordance with the present invention, contains the oil constituent, which contains the solid or semisolid non-polar hydrocarbon oil constituent, and the particles.

The term "fine wrinkles" as used herein means the comparatively shallow wrinkles occurring about the eyes or the mouths due to the drying of the skin and embraces the fine wrinkles occurring due to atopic dermatitis.

Also, the term "fine wrinkle improving" or "improving fine wrinkles" as used herein means that the fine wrinkles having been formed are diminished and that the formation of the fine wrinkles is prevented.

In the present invention, the blending proportion of the oil constituent contained in the oil based preparation for diminishing fine wrinkles falls within the range of 70% by mass to 85% by mass with respect to the total quantity of the preparation. . If the blending proportion of the oil constituent is lower than 50% by mass, high occlusion characteristics will not be obtained. If the blending proportion of the oil constituent is higher than 95% by mass, the usability will become bad.

The solid or semisolid non-polar hydrocarbon oil constituent employed in the present invention may be selected from a wide variety of oil constituents, which are solid or semisolid at normal temperatures. Examples of the solid or semisolid non-polar hydrocarbon oil constituents include solid paraffin, micro-crystalline wax, ceresine, bareco wax, polyethylene wax, hardened oil, polyethylene powder and petrolatum. In the present invention, the solid or semisolid non-polar hydrocarbon oil constituent acts to efficiently prevent water from being vaporized and lost from the skin, to retain water at the surface of the skin, and to prevent the skin from drying. Therefore, the solid or semisolid non-polar hydrocarbon oil constituent employed should preferably have high occlusion characteristics. The solid or semisolid non-polar hydrocarbon oil constituent should preferably be such as micro-crystalline wax, polyethylene wax, or petrolatum.

In the present invention, the blending proportion of the solid or semisolid non-polar hydrocarbon oil constituent is of at least 30% by mass with respect to the total quantity of the oil constituent. If the blending proportion of the solid or semisolid non-polar hydrocarbon oil constituent is lower than 10% by mass, high occlusion characteristics will not be obtained. A preferable blending proportion of the solid or semisolid oil constituent is not limited and may vary in accordance with the kind of the oil constituent employed, the oil constituent combination, or the like. Particularly, the solid or semisolid non-polar hydrocarbon oil constituent should preferably be contained in a proportion of at least 40% by mass with respect to the total quantity of the oil constituent. In cases where the solid or semisolid non-polar hydrocarbon oil constituent is blended at a high blending proportion, particularly high occlusion characteristics and high-temperature stability are capable of being obtained.

In the present invention, from the view point of the usability, the oil constituent should preferably contain the volatile oil constituent. In cases where the volatile oil constituent is blended, the coating and extension and the sticky feeling of the coating composition are capable of being improved even further.

The term "volatile oil constituent" as used herein means the oil constituent, which has the volatility at the room temperature (25°C). In the present invention, the volatile oil constituent may be selected from a wide variety of oil constituents, with which the purposes of the present invention are capable of being accomplished. Examples of the volatile oil constituents include isoparaffin type hydrocarbon oils having a low boiling temperature (a boiling temperature of at most 260°C at the normal pressure) and silicone oils having a low boiling temperature.

Specifically, the isoparaffin type hydrocarbon oils having a low boiling temperature are commercially available under the trade names of Isopar A, Isopar C, Isopar E, Isopar G, Isopar H, Isopar K, Isopar L, and Isopar M (supplied by Exxon Co.); Shellsole 71 (supplied by Shell Co.) ; Soltrol 100, Soltrol 130, and Soltrol 220 (supplied by Phillip Co.).

Examples of the preferable silicone oils having a low boiling temperature include hexamethylcyclotrisiloxane; octarnethyltetracyclosiloxane (e.g., Execol D-4, supplied by Shin-Etsu Silicone Co.); SH244, SH344 (supplied by Dow Corning Toray Co., Ltd.) ; decamethylcyclopentasiloxane (e.g., Execol D-5, supplied by Shin-Etsu Silicone C.); SH245, DC345 (supplied by Dow Corning Toray Co., Ltd.); dodecamethylcyclohexasiloxane (e.g., DC246, supplied by Dow Corning Toray Co., Ltd.): and tetradecamethylcycloheptasiloxane. Particularly, for the advantages of having good usability improving effects, decamethylcyclopentasiloxane should preferably be blended as the volatile oil constituent.

In the present invention, one kind of the volatile oil constituent may be used alone. Alternatively, two or more kinds of the volatile oil constituents may be used in combination. The blending proportion of the volatile oil constituent should preferably fall within the range of 5% by mass to 50% by mass with respect to the total quantity of the oil constituent. The blending proportion of the volatile oil constituent should more preferably fall within the range of 10% by mass to 40% by mass with respect to the total quantity of the oil constituent, and should most preferably fall within the range of 15% by mass to 30% by mass with respect to the total quantity of the oil constituent.

Also, a liquid oil constituent, which is liquid at the room temperature (25°C), may be blended, such that the purposes of the present invention may be accomplished. Particularly, since the solid oil constituent itself is markedly hard and exhibits a low usability, the liquid oil constituent should preferably be contained together with the solid oil constituent. Examples of the liquid oil constituents include non-polar hydrocarbon oils, such as liquid paraffin, and squalane; fats and oils, such as olive oil, macadamia nut oil, and jojoba oil; higher fatty acids, such as oleic acid, tall oil fatty acid, and isostearic acid; higher alcohols, such as lauryl alcohol, oleyl alcohol, isostearyl alcohol, and octyldodecanol; esters, such as isocetyl isostearate, myristyl myristate, and isopropyl palmitate; chain polysiloxanes, such as dimethylpolysiloxane, methylphenylpolysiloxane, and methylhydrogenpolysiloxane; ultraviolet light absorbers, such as benzophenone derivatives; and perfumes. Particularly, from the view point of the occlusion characteristics, the non-polar hydrocarbon type of liquid oil, such as liquid paraffin or squalane, should preferably be blended. One kind of the liquid oil constituent enumerated above may be used alone. Alternatively, two to more kinds of the liquid oil constituents enumerated above may be used in combination. Also, the blending proportion of the liquid oil constituent is not limited and may vary in accordance with the kind of the oil constituent employed and the oil constituent combination. The blending proportion of the liquid oil constituent should preferably fall within the range of 10% by mass to 90% by mass with respect to the total quantity of the oil constituent. The blending proportion of the liquid oil constituent should more preferably fall within the range of 20% by mass to 80% by mass with respect to the total quantity of the oil constituent, and should most preferably fall within the range of 30% by mass to 70% by mass with respect to the total quantity of the oil constituent.

The particles employed in the present invention may be selected from a wide variety of kinds of particles, with which the purposes of the present invention are capable of being accomplished. In the present invention, it is possible to use, for example, organic particles, such as polyethylene particles, polymethyl methacrylate particles, polystyrene particles, nylon particles, silicone resin particles, silicone rubber particles, silicone resin-coated silicone rubber particles, and polyurethane particles; and inorganic particles, such as silica particles, talcparticles, kaolin particles, and sericite particles. No limitation is imposed upon the particle shapes. However, spherical particles are blended for their advantages in that the coating and extension are capable of being improved, and in that the unevenness of the skin is capable of being blurred and rendered imperceptible by virtue of the light scatteringeffects. Particularly, from the viewpoint of suppressing the powdery feeling, elastic spherical particles are used. Examples of the elastic spherical particles include spherical silicone rubber particles, spherical silicone resin-coated rubber particles, and spherical polyurethane particles. Among the above-enumerated elastic spherical particles, the elastic spherical silicone type particles are used for their high effects of improving the sticky feeling. The elastic spherical silicone type particles, such as spherical silicone rubberparticles, silicone resin-coated silicone rubber particles, and zinc oxide-coated, silicone resin-coated silicone rubber particles, are blended, the coating and extension become smooth, the sticky feeling and the powdery feeling are eliminated, and markedly good usability is obtained. The mean particle diameter of the aforesaid particles should preferably fall within the range of $1\mu m$ to $50\mu m$. The particles described above are available commercially. In the present invention, for example, Trefil E506W (supplied by Dow Corning Toray Co., Ltd.) may be used appropriately as the silicone rubber particles, and Silicone Powder KSP100 (supplied by Shin-Etsu Chemical Co., Ltd.) maybe used appropriately as the silicone resin-coated silicone rubber particles.

Spherical silica particles are used in a proportion falling within the range of, for example, approximately 1% by mass to approximately 5%bymass in conjunction with the aforesaid elastic spherical particles or the aforesaid elastic spherical silicone type particles, the high-temperature vertical storage stability is enhanced. Ordinarily, the oil based preparation for diminishing fine wrinkles as used in accordance with the present invention is stored in the horizontal direction. However, it is more preferable that, besides the storage stability in the horizontal storage direction, the storage stability in the vertical storage direction is also guaranteed. Particularly, good effects have been obtained where porous spherical silica particles are used. In the present invention, for example, SILDEX L-51 (supplied by Asahi Glass Co., Ltd.) or Spherical Silica P-1500 (supplied by JGC Catalysts and Chemicals Ltd.) may be used appropriately.

Further, various kinds of the extender pigments may be coated with particles having a refractive index of at least 1.6, such as barium sulfate (refractive index: 1.64), zinc oxide (refractive index: 2.0), and titanium oxide (rutile type) (refractive index: 2.7), or may be formed as composite particles by use of the aforesaid particles having a refractive index of at least 1.6. Alternatively, part of the particles may be subjected to simple mixing with the aforesaid particles having a refractive index of at least 1.6. In this manner, it is possible to obtain high color heterogeneity concealing effects.

In cases where part of the particles are subjected to the simple mixing with the aforesaid particles having a refractive index of at least 1.6, the aforesaid particles having a refractive index of at least 1.6 should preferably be blended in a proportion of approximately 0.5% by mass to approximately 3% by mass in the oil based preparation for diminishing fine wrinkles in accordance with the present invention. If the proportion of the aforesaid particles having a refractive index of at least 1.6 in the oil based preparation for diminishing fine wrinkles is higher than 3% by mass, at the time at which the oil based preparation is coated onto the skin, the color of the skin will become markedly white and an unnatural feeling will be given.

In cases where the particles coated with the aforesaid particles having a refractive index of at least 1.6 or the composite particles formed by use of the aforesaid particles having a refractive index of at least 1.6 are blended, the particles coated with the aforesaid particles having a refractive index of at least 1.6 or the composite particles formed by use of the aforesaid particles having a refractive index of at least 1.6 maybe blended in a proportion of approximately 5% by mass to approximately 30% by mass in the oil based preparation for diminishing fine wrinkles, which is used in accordance with the present invention. In such cases, it is possible to obtain high color heterogeneity concealing effects . In cases where the particles coated with the aforesaid particles having a refractive index of at least 1.6 or the composite particles formed by use of the aforesaid particles having a refractive index of at least 1.6 are blended, it is possible to obtain the color heterogeneity concealing effects higher than in cases where part of the particles are subjected to the simple mixing with the aforesaid particles having a refractive index of at least 1.6. Also, in cases where the particles coated with the aforesaid particles having a refractive index of at least 1.6 or the composite particles formed by use of the aforesaidparticles having a refractive index of at least 1.6 are blended, at the time at which the oil based preparation for diminishing fine wrinkles is coated onto the skin, it is possible to obtain a skin feeling more beautiful than in cases where part of the particles are subjected to the simple mixing with the aforesaid particles having a refractive index of at least 1.6. Therefore, it is more preferable to blend the particles coated with the aforesaid particles having a refractive index of at least 1.6 or the composite particles formed by use of the aforesaid particles having a refractive index of at least 1.6.

In the oil based preparation used for diminishing fine wrinkles two or more kinds of the particles may be blended. The blending proportion of the particles in the oil based preparation for diminishing fine wrinkles should fall within the range of 15% by mass to 30% by mass with respect to the total quantity of the preparation. If the blending proportion of the particles in the oil based preparation for diminishing fine wrinkles used in accordance with the present invention is lower than 5% by mass with respect to the total quantity of the preparation, the oily sheen with the oil constituent, the sticky feeling with the oil constituent, and the like, will not be improved sufficiently. If the blending proportion of the particles in the oil based preparation for diminishing fine wrinkles is higher than 50% by mass with respect to the total quantity of the preparation, the occlusion characteristics will become bad.

The blending proportion of the spherical particles ordinarily falls within the range of 30% by mass to 100% by mass with respect to the total quantity of the particles, and should preferably fall within the range of 50% by mass to 100% by mass with respect to the total quantity of the particles.

In the present invention, the oil based preparation used for external use for skins has the occlusion characteristics of at least 50%. In order for the fine wrinkles to be improved more efficiently, the oil based preparation for diminishing fine wrinkles has the occlusion characteristics higher than 50%, for example, the occlusion characteristics of at least 60%. The oil based preparation for diminishing fine wrinkles used in accordance with the present invention should more preferably have the occlusion characteristics of at least 70%, and should most preferably have the occlusion characteristics of at least 80%. The occlusion characteristics may be calculated in the manner described below. Specifically, a sample is applied (at a rate of $2.5 mg/cm^2$) to an internal site of a human forearm. At a stage one hour after the sample has thus been applied, the transepidermal water loss (TEWL) is measured with a water loss meter, such as Tewameter TM210 (supplied by Courage+Khazaka), MEECO (supplied by Meeco, Warrington, PA, USA), Vapometer, or TEWA meter (supplied by Delfin Technologies Ltd., Kuopio, Finland). Thereafter, the occlusion characteristics are calculated with the formula shown below.

```
Occlusion characteristics (%)

= (1-TEWL (with sample) / TEWL (without sample)) × 100
```

The use of the oil based preparation for diminishing fine wrinkles in accordance with the present invention may embrace cosmetic preparations, pharmaceutical preparations, quasi-drugs, and the like. Also, the oil based preparation used for diminishing fine wrinkles may take on a wide variety of preparation forms, such as an ointment type, a paste type, and a skin cream type.

When necessary, besides the essential constituents described above, the oil based preparation for diminishing fine wrinkles may also conta in other arbitrary constituents, which are ordinarily used in oil based preparations for external use for skins, such as the cosmetic preparations and the pharmaceutical preparations, within a range such that the effects of the present invention may not be affected adversely. Examples of the other arbitrary constituents described above include a surface active agent, an ultraviolet light absorber, a perfume, an anti-oxidant, an antiseptic agent, a mildewproofing agent, an extender pigment, a coloring material (such as a coloring pigment), and a pH regulating agent.

Also, when necessary, insofar as the desired effects of the present invention are not affected adversely, various medicines having the wrinkle improving effects, moisturizing agents, and the like, may be blended with the oil based preparation for diminishing fine wrinkles . Examples of the aforesaid medicines, the aforesaid moisturizing agents, and the like, include vitamin A, vitamin A palmitate, vitamin A acetate, $\alpha$-tocopherol, $\beta$-tocopherol, $\delta$-tocopherol, vitaminE acetate, vitamin D, BHT, octylmethoxy cinnamate, octacrylene, 2,2'-hydroxy-5-methylphenyl benzotriazole, 2-(2'-hydroxy-5'-t-octylphenyl) benzotriazole, 2-(2'-hydroxy-5'-methylphenyl) benzotriazole, 2-(2-hydroxy-4-(2-ethylhexyl)phenoxy)-2H-benzotriazole, 4-methoxy-4'-t-butyldibenzoylmethane, 5-(3,3-dimethyl-2-norbornylidene)-3-pentan-2-one, 2,4,6-tris [p-((2'-ethylhexyl) oxycarbonyl) aniline] 1, 3, 5-triazine, and an alkylaryl-1,3-propanedione silicone derivative.

From the view point of the occlusion characteristics, the oil based preparation used for diminishing fine wrinkles should preferably be substantially free from water and water-soluble constituents. However, the oil based preparation for diminishing fine wrinkles may take on the form of W/O type emulsions containing a small amount of water, such that the purposes of the present invention are capable of being accomplished. The proportion of water and water-soluble constituents contained in the oil based preparation used for diminishing fine wrinkles is at most 1% by mass.

In the cosmetic method in accordance with the present invention, no limitation is imposed upon the number of times of the application of the oil based preparation for diminishing fine wrinkles to the skin, the period of time for which the oil based preparation for diminishing fine wrinkles is applied to the skin. For example, even in the cases of the application for a short period of time (e.g., one night application), the skin becomes soft and full by virtue of water having been supported on the skin in the next morning, and the fine wrinkle improving effects are obtained. The oil based preparation for diminishing fine wrinkles should preferably be used successively, for example, for one week, two weeks, one month, or a longer period of time. By the successive use, high fine wrinkle improving effects are obtained.

The oil based preparation for diminishing fine wrinkles, which is used in accordance with the present invention may be used alone. Alternatively, the oil based preparation for diminishing fine wrinkles may be used in combination with other skin care preparations or make-up preparations. For example, in cases where the oil based preparation for diminishing fine wrinkles is used at the last stage of skin care, high wrinkle improving effects are obtained. Also, the oil based preparation for diminishing fine wrinkles may be applied to the skin before make-up is performed.

Further, in the cosmetic method in accordance with the present invention, the site of the skin to which the oil based preparation for diminishing fine wrinkles is applied, and the manner in which the oil based preparation for diminishing fine wrinkles is applied are not limited may be set arbitrarily insofar as the effects of the present invention are obtained.

The cosmetic method in accordance with the present invention may be applied in various ways relevant to the improvement of the fine wrinkles.

The present invention will further be illustrated by the following non-limitative examples.

Examples

1. Study of occlusion characteristics

Preparations for external use were prepared in accordance with recipes (Test Examples 1 to 13) listed in Table 1 below. With respect to each of the samples, the occlusion characteristics and the fine wrinkle improving effects were evaluated with the methods described below.

Evaluation of occlusion characteristics

The occlusion characteristics of each of the preparations for external use were evaluated in accordance with the results of the measurement of the transepidermal water loss (TEWL). Specifically, as for each special panel of ten persons for each sample, the sample was applied (at a rate of $2.5mg/cm^2$) to an internal site of the human forearm. At a stage one hour after the sample had thus been applied, the transepidermal water loss (TEWL) was measured with a water loss meter (Tewameter TM210, supplied by Courage+Khazaka). Thereafter, the occlusion characteristics were calculated with the formula shown below.

```
Occlusion characteristics (%)
= (1-TEWL (with sample) / TEWL (without sample)) × 100
```

Evaluation of fine wrinkle improving effects

As for each panel having fine wrinkles (ten persons for each sample), the sample was applied (at a rate of $2mg/cm^2$) before sleeping at night. After one night application and one month application, the fine wrinkle improving effects of the preparation for external use were evaluated with the evaluation criteria shown below.

(Evaluation criteria after one night application)

A: At least seven persons of the panel answered that the fine wrinkles became soft and full and became imperceptible.

B: Four to six persons of the panel answered that the fine wrinkles became soft and full and became imperceptible.
C: Two to three persons of the panel answered that the fine wrinkles became soft and full and became imperceptible.
D: At most one person of the panel answered that the fine wrinkles became soft and full and became imperceptible.

(Evaluation criteria after one month application)

A: At least seven persons of the panel answered that the fine wrinkles had been improved.
B: Four to six persons of the panel answered that the fine wrinkles had been improved.
C: Two to three persons of the panel answered that the fine wrinkles had been improved.
D: At most one person of the panel answered that the fine wrinkles had been improved.

The results shown in Table 1 were obtained.

Table 1

| Recipe | Test Ex. 1 | Test Ex. 2 | Test Ex. 3 | Test Ex. 4 | Test Ex. 5 | Test Ex. 6 |
|---|---|---|---|---|---|---|
| Petrolatum (non-polar hydrocarbon oil, semisolid) | 100 | 80 | 65 | 50 | 30 | 15 |
| Micro-crystalline wax (non-polar hydrocarbon oil, solid) | 0 | 0 | 0 | 10 | 20 | 20 |
| Liquid paraffin (non-polar hydrocarbon oil, liquid) | 0 | 20 | 35 | 40 | 50 | 65 |
| Dimethylpolysiloxane (silicone oil, liquid) | - | - | - | - | - | - |
| Trioctanoin (polar hydrocarbon oil, liquid) | - | - | - | - | - | - |
| Evaluation | | | | | | |
| Occlusion characteristics (%) | 80% | 60% | 60% | 60% | 50% | 50% |
| Fine wrinkle improving effects (after one night application) | A | B | B | B | C | C |
| Fine wrinkle improving effects (after one month application) | A | B | B | B | C | C |

EP 2 042 159 B1

Table 1 - continued

| Recipe | Test Ex. 7 | Test Ex. 8 | Test Ex. 9 | Test Ex. 10 | Test Ex. 11 | Test Ex. 12 | Test Ex. 13 |
|---|---|---|---|---|---|---|---|
| Petrolatum (non-polar hydrocarbon oil, semisolid) | 0 | 15 | 0 | 85 | 85 | 65 | 75 |
| Micro-crystalline wax (non-polar hydrocarbon oil, solid) | 20 | 35 | 40 | 0 | 0 | 0 | 0 |
| Liquid paraffin (non-polar hydrocarbon oil, liquid) | 80 | 50 | 60 | 0 | 0 | 0 | 0 |
| Dimethylpolysiloxane (silicone oil, liquid) | -- | -- | -- | 0 | 15 | 0 | 25 |
| Trioctanoin (polar hydrocarbon oil, liquid) | -- | -- | -- | 15 | 0 | 35 | 0 |
| Evaluation | | | | | | | |
| Occlusion characteristics (%) | 50% | 50% | 50% | 50% | 50% | 40% | 40% |
| Fine wrinkle improving effects (after one night application) | C | C | C | C | C | D | D |
| Fine wrinkle improving effects (after one month application) | C | C | C | C | C | D | D |

EP 2 042 159 B1

With each of the oil based preparations for diminishing fine wrinkles having the high occlusion characteristics of at least 50% (Test Examples 1 to 11), after the one night application and after the one month application, the soft and full feeling was obtained, and the fine wrinkles were improved. With each of the oil based preparations for diminishing fine wrinkles having the occlusion characteristics lower than 50% (Test Examples 12 and 13), the fine wrinkle improving effects were not obtained even after the one month application.

2. <u>Study of improvement in usability</u>

Since the usability was bad with the oily constituent alone due to bad spreadability and the occurrence of a sticky feeling, the study was made to blend various particles for improving the usability such that the occlusion characteristics of the oil based preparations for diminishing fine wrinkles might not be affected adversely.

Various kinds of the particles were blended with the oil constituents in accordance with the recipes shown in Table 2 below, and oil based preparations for diminishing fine wrinkles were prepared. With respect to each of the samples, the occlusion characteristics and the fine wrinkle improving effects were evaluated in the same manner as that described above. Also, the usability was evaluated in the manner described below.

<u>Evacuation of usability</u>

The evaluation of the usability (the sticky feeling, the spreadability, and the powdery feeling) was made with functional tests by special panels. Specifically, each of the samples was used by each special panel of 10 females, and the usability was evaluated with the evaluation criteria shown below.

(Sticky feeling)

A: At least seven females of the panel answered that the sticky feeling was not given.
B: Three to six females of the panel answered that the sticky feeling was not given.
C: At most two females of the panel answered that the sticky feeling was not given.

(Spreadability)

A: At least seven females of the panel answered that the spreadability was excellent.
B: Three to six females of the panel answered that the spreadability was excellent.
C: At most two females of the panel answered that the spreadability was excellent.

(Powdery feeling)

A: At least seven females of the panel answered that the powdery feeling was not given.
B: Three to six females of the panel answered that the powdery feeling was not given.
C: At most two females of the panel answered that the powdery feeling was not given.

The results shown in Table 2 were obtained.

Table 2

| Recipe | Ex. 1 | Ex. 2 | Ex. 3 | Ex. 4 | Ex. 5 | Ex. 6 |
|---|---|---|---|---|---|---|
| Petrolatum (*1) | 80 | 25 | 25 | 25 | 20 | 25 |
| Micro-crystalline wax (*2) | – | 15 | 15 | 15 | 12 | 15 |
| Liquid paraffin (*3) | – | 40 | 40 | 40 | 32 | 40 |
| Decamethylcyclopentasiloxane (*4) | – | – | – | – | 20 | – |
| Spherical silicone rubber particles (*5) | 20 | – | – | – | – | – |
| Coated silicone rubber particles (*6) | – | 20 | 17 | 18 | 16 | – |
| Spherical silicone resin particles (*7) | – | – | – | – | – | 20 |
| Silica (spherical) | – | – | 3 | – | – | – |
| Nylon (spherical) | – | – | – | – | – | – |
| Talc (*8) | – | – | – | – | – | – |
| Zinc oxide (refractive index: 2.0) | – | – | – | 2 | – | – |
| Evaluation | | | | | | |
| Occlusion characteristics (%) | 70% | 70% | 70% | 70% | 60% | 60% |
| Fine wrinkle improving effects (after one night application) | A | A | A | A | B | B |
| Fine wrinkle improving effects (after one month application) | A | A | A | A | B | B |
| Sticky feeling | A | A | A | A | A | A |
| Spreadability | B | B | B | B | A | B |
| Powdery feeling | A | A | A | A | A | B |

13

Table 2 - continued

| Recipe | Ex. 7 | Ex. 8 | Ex. 9 | Comp. 1 | Comp. 2 | Comp. 3 | Comp. 4 |
|---|---|---|---|---|---|---|---|
| Petrolatum (*1) | 75 | 75 | 80 | 100 | 30 | 50 | 30 |
| Micro-crystalline wax (*2) | – | – | – | – | 10 | – | – |
| Liquid paraffin (*3) | – | – | – | – | 60 | 49 | 5 |
| Decamethylcyclopentasiloxane (*4) | – | – | – | – | – | – | – |
| Spherical silicone rubber particles (*5) | – | – | – | – | – | 1 | 65 |
| Coated silicone rubber particles (*6) | – | – | – | – | – | – | – |
| Spherical silicone resin particles (*7) | – | – | – | – | – | – | – |
| Silica (spherical) | 25 | – | – | – | – | – | – |
| Nylon (spherical) | – | 25 | – | – | – | – | – |
| Talc (*8) | – | – | 20 | – | – | – | – |
| Zinc oxide (refractive index: 2.0) | – | – | – | – | – | – | – |
| Evaluation | | | | | | | |
| Occlusion characteristics (%) | 60% | 60% | 60% | 80% | 50% | 60% | 40% |
| Fine wrinkle improving effects (after one night application) | B | B | B | A | C | B | D |
| Fine wrinkle improving effects (after one month application) | B | B | B | A | C | B | D |
| Sticky feeling | B | B | B | C | C | C | A |
| Spreadability | B | B | B | C | C | C | A |
| Powdery feeling | B | B | C | A | A | A | C |

EP 2 042 159 B1

Table 2 – continued

*1: Non-polar hydrocarbon oil, semisolid
*2: Non-polar hydrocarbon oil, solid
*3: Non-polar hydrocarbon oil, liquid
*4: Silicone oil, non-polar oil, volatile
*5: Spherical, elastic, refractive index: 1.4
*6: Zinc oxide (refractive index: 2.0)-coated silicone resin-coated silicone rubber particles (spherical, elastic)
*7: Spherical, refractive index: 1.4
*8: Plate-shaped, refractive index: 1.57

In each of Examples 1 to 9, wherein the particles were blended in a proportion of at most 50% by mass, the sticky feeling and the spreadability were capable of being improved markedly, such that the occlusion characteristics (and consequently the fine wrinkle improving effects) might not be affected adversely. Particularly, in each of Examples 1 to 8, wherein the spherical particles were blended, the coating and extension smoothness was good. Also, in each of Examples 1 to 5, wherein the spherical particles were the elastic spherical particles, the powdery feeling was not given, and the usability was enhanced even further. Further, in Example 5, wherein the volatile liquid oil constituent was blended, the spreadability was enhanced even further.

Furthermore, though not illustrated by the data, in Example 3, wherein the elastic spherical particles and the spherical silica particles were blended, the stability at the time at which the preparation for diminishing fine wrinkles was stored in the vertical direction at a high temperature (50°C) for one month was good. The preparation for external use of Example 2, wherein only the elastic spherical particles were blended, was stable in the state of the horizontal direction in which the oil based preparation for diminishing fine wrinkles is ordinarily stored. However, with the preparation for diminishing fine wrinkles of Example 2, slight oil floating was observed in cases where the preparation was stored in the vertical direction at a high temperature (50°C) for one month. In Example 3, wherein the spherical silica particles were blended together with the elastic spherical particles, oil floating was not observed at the time at which the preparation for diminishing fine wrinkles was stored in the vertical direction at a high temperature (50°C) for one month. Ordinarily, the oil based preparation used for diminishing fine wrinkles is not stored in the vertical direction, and no problems will occur in cases where the stability of the storage in the horizontal direction is good. However, in cases where emergency, or the like, is taken into consideration, the preparation for diminishing fine wrinkles should preferably also have the storage stability in the vertical direction.

Also, in cases where the particles having a refractive index of at least 1. 6 or the particles having been coated with the particles having a refractive index of at least 1.6 were blended (in Examples 2 to 5), the protrusion and recesses of the skin were capable of being rendered imperceptible, and good color heterogeneity concealing effects were obtained.

In Comparative Examples 1 and 2, in which the particles were not blended, and in Comparative Example 3, in which the blending proportion of the particles was lower than 5% by mass, the sticky feeling occurred, and the spreadability was markedly bad. Also, in Comparative Example 4, in which the blending proportion of the particles was higher than 50% by mass, the occlusion characteristics were low, and the fine wrinkle improving effects were not obtained.

**Claims**

1. Use of an oil based preparation for diminishing fine wrinkles, wherein the oil based preparation containing:

   i) 70% by mass to 85% by mass of an oil constituent, which contains a solid or semisolid non-polar hydrocarbon oil in a proportion of at least 30% by mass with respect to a total quantity of the oil constituent, and
   ii) 15% by mass to 30% by mass of particles, which comprise elastic spherical silicone type particles and spherical silica particles,

   wherein the oil based preparation has occlusion characteristics of at least 50%, and the proportion of water and water-soluble constituents contained in the oil based preparation is at most 1 % by mass.

2. Use as defined in Claim 1, wherein said elastic spherical silicone type particles comprise at least one member selected from the group consisting of spherical silicone rubber particles, silicone resin-coated silicone rubber particles, and zinc oxide-coated, silicone resin-coated silicone rubber particles.

3. Use as defined in Claim 1 or 2, wherein said solid or semisolid non-polar hydrocarbon oil comprises petrolatum and/or micro-crystalline wax.

4. Use as defined in Claim 1, 2 or 3, wherein the oil constituent contains liquid non-polar hydrocarbon oil.

5. Use as defined in Claim 1, 2, 3 or 4 wherein the oil constituent contains a volatile oil constituent.

6. A cosmetic method for diminishing fine wrinkles, wherein an oil based preparation for external use for skins is applied onto a skin, the oil based preparation containing:

   i) 70% by mass to 85% by mass of an oil constituent, which contains a solid or semisolid non-polar hydrocarbon oil in a proportion of at least 30% by mass with respect to a total quantity of the oil constituent, and
   ii) 15% by mass to 30% by mass of particles, which comprise elastic spherical silicone type particles and spherical silica particles,

   wherein the oil based preparation has occlusion characteristics of at least 50%, and the proportion of water and water-soluble constituents contained in the oil based preparation is at most 1 % by mass.

7. A cosmetic method as defined in Claim 6, wherein said elastic spherical silicone type particles comprise at least one member selected from the group consisting of spherical silicone rubber particles, silicone resin-coated silicone rubber particles, and zinc oxide-coated, silicone resin-coated silicone rubber particles.

8. A cosmetic method as defined in Claim 6 or 7, wherein said solid or semisolid non-polar hydrocarbon oil comprises petrolatum and/or micro-crystalline wax.

9. A cosmetic method as defined in Claim 6,7 or 8, wherein the oil constituent contains liquid non-polar hydrocarbon oil.

10. A cosmetic method as defined in Claim 6, 7, 8 or 9 wherein the oil constituent contains a volatile oil constituent.

**Patentansprüche**

1. Verwendung einer Zubereitung auf Ölbasis zur Verminderung feiner Fältchen, wobei die Zubereitung auf Ölbasis enthält:

   i) 70 Massenprozent bis 85 Massenprozent eines Ölbestandteils, der ein festes oder halbfestes nicht polares Kohlenwasserstofföl in einem Anteil von wenigstens 30 Massenprozent in Bezug auf eine Gesamtmenge des Ölbestandteils enthält, und
   ii) 15 Massenprozent mit 30 Massenprozent an Teilchen, die elastische kugelförmige silikonartige Teilchen und kugelförmige Silikateilchen umfassen,

   wobei die Zubereitung auf Ölbasis Okklusionseigenschaften von wenigstens 50% aufweist und der Anteil an Wasser und wasserlöslichen Bestandteilen, die in der Zubereitung auf Ölbasis enthalten sind, höchstens 1 Massenprozent ist.

2. Verwendung nach Anspruch 1, wobei die elastischen kugelförmigen silikonartigen Teilchen wenigstens ein Element umfassen, das ausgewählt ist aus der Gruppe bestehend aus kugelförmigen Silikonkautschukteilchen, Silikonharz-beschichteten Silikonkautschukteilchen und Zinkoxid-beschichteten, Silikonharz-beschichteten Silikonkautschukteilchen.

3. Verwendung nach Anspruch 1 oder 2, wobei das feste oder halbfeste nicht polare Kohlenwasserstofföl Petrolatum und/oder mikrokristallines Wachs umfasst.

4. Verwendung nach Anspruch 1, 2 oder 3, wobei der Ölbestandteil flüssiges nicht polares Kohlenwasserstofföl enthält.

5. Verwendung nach Anspruch 1, 2, 3 oder 4, wobei der Ölbestandteil einen flüchtigen Ölbestandteil enthält.

6. Kosmetisches Verfahren zum Vermindern von feinen Fältchen, wobei eine Zubereitung auf Ölbasis zur äußeren Anwendung für Haut auf eine Haut aufgetragen wird, wobei die Zubereitung auf Ölbasis enthält:

   i) 70 Massenprozent bis 85 Masseprozent eines Ölbestandteils, der ein festes oder halbfestes nicht polares Kohlenwasserstofföl in einem Anteil von wenigstens 30 Massenprozent in Bezug auf eine Gesamtmenge des Ölbestandteil enthält, und
   ii) 15 Massenprozent bis 30 Massenprozent von Teilchen, die elastische kugelförmige silikonartige Teilchen und kugelförmige Silikateilchen umfassen,

   wobei die Zubereitung auf Ölbasis Okklusionseigenschaften von wenigstens 50% aufweist und der Anteil an Wasser und wasserlöslichen Bestandteilen, die in der Zubereitung auf Ölbasis enthalten sind, höchstens 1 Massenprozent ist.

7. Kosmetisches Verfahren nach Anspruch 6, wobei die elastischen kugelförmigen silikonartigen Teilchen wenigstens ein Element umfassen, das ausgewählt ist aus der Gruppe bestehend aus kugelförmigen Silikonkautschukteilchen, Silikonharz-beschichteten Silikonkautschukteilchen und Zinkoxid-beschichteten, Silikonharz-beschichteten Silikonkautschukteilchen.

8. Kosmetisches Verfahren nach Anspruch 6 oder 7, wobei das feste oder halbfeste nicht polare Kohlenwasserstofföl Petrolatum und/oder mikrokristallines Wachs umfasst.

9. Kosmetisches Verfahren nach Anspruch 6, 7 oder 8, wobei der Ölbestandteil flüssiges nicht polares Kohlenwasserstofföl enthält.

10. Kosmetisches Verfahren nach Anspruch 6, 7. 8 oder 9, wobei der Ölbestandteil einen flüchtigen Ölbestandteil enthält.

**Revendications**

1. Utilisation d'une préparation à base d'huile pour la réduction des rides fines, dans laquelle la préparation à base d'huile contient :

   i) de 70 % en poids à 85 % en poids d'un constituant huileux, qui contient une huile hydrocarbonée non polaire, solide ou semi-solide, dans une proportion d'au moins 30 % en poids par rapport à une quantité totale du constituant huileux, et

ii) de 15 % en poids à 30 % en poids de particules, qui comprennent des particules sphériques élastiques de type silicone et des particules sphériques de silice,

dans laquelle la préparation à base d'huile a des caractéristiques d'occlusion d'au moins 50 % et la proportion d'eau et de constituants solubles dans l'eau contenus dans la préparation à base d'huile s'élève au plus à 1 % en poids.

2. Utilisation selon la revendication 1, dans laquelle lesdites particules sphériques élastiques de type silicone comprennent au moins un élément choisi dans le groupe comprenant des particules sphériques de caoutchouc de silicone, des particules de caoutchouc de silicone revêtues de résine de silicone et des particules de caoutchouc de silicone revêtues de résine de silicone, revêtues d'oxyde de zinc.

3. Utilisation selon la revendication 1 ou 2, dans laquelle l'huile hydrocarbonée non polaire, solide ou semi-solide, comprend du pétrolatum et/ou de la cire microcristalline.

4. Utilisation selon la revendication 1, 2 ou 3, dans laquelle le constituant huileux contient de l'huile hydrocarbonée non polaire liquide.

5. Utilisation selon la revendication 1, 2, 3 ou 4, dans laquelle le constituant huileux contient un constituant huileux volatil.

6. Procédé cosmétique pour réduire les rides fines, dans lequel une préparation à base d'huile pour un usage externe pour la peau est appliquée sur une peau, la préparation à base d'huile contenant :

i) de 70 % en poids à 85 % en poids d'un constituant huileux, qui contient une huile hydrocarbonée non polaire, solide ou semi-solide, dans une proportion d'au moins 30 % en poids par rapport à une quantité totale du constituant huileux, et

ii) de 15 % en poids à 30 % en poids de particules, qui comprennent des particules sphériques élastiques de type silicone et des particules sphériques de silice,

dans lequel la préparation à base d'huile a des caractéristiques d'occlusion d'au moins 50 % et la proportion d'eau et de constituants solubles dans l'eau contenus dans la préparation à base d'huile s'élève au plus à 1 % en poids.

7. Procédé cosmétique selon la revendication 6, dans lequel lesdites particules sphériques élastiques de type silicone comprennent au moins un élément choisi dans le groupe comprenant des particules sphériques de caoutchouc de silicone, des particules de caoutchouc de silicone revêtues de résine de silicone et des particules de caoutchouc de silicone revêtues de résine de silicone, revêtues d'oxyde de zinc.

8. Procédé cosmétique selon la revendication 6 ou 7, dans lequel ladite huile hydrocarbonée non polaire, solide ou semi-solide, comprend du pétrolatum et/ou de la cire microcristalline.

9. Procédé cosmétique selon la revendication 6, 7 ou 8, dans lequel le constituant huileux contient de l'huile hydrocarbonée non polaire liquide.

10. Procédé cosmétique selon la revendication 6, 7, 8 ou 9, dans lequel le constituant huileux contient un constituant huileux volatil.

**EP 2 042 159 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002047138 A
- JP 2003012461 A
- JP 2000016919 A
- JP 2002080338 A
- JP 2000026232 A
- JP 2003002814 A
- JP 2003026608 A
- EP 1839647 A1
- US 5945095 A
- WO 0205751 A1
- US 5919468 A
- EP 1627629 A1